Europäisches Patentamt

European Patent Office

Office européen des brevets

(19)

(11) **EP 0 917 909 B1**

(12) **EUROPÄISCHE PATENTSCHRIFT**

(45) Veröffentlichungstag und Bekanntmachung des Hinweises auf die Patenterteilung:
**15.09.1999 Patentblatt 1999/37**

(21) Anmeldenummer: **98111180.0**

(22) Anmeldetag: **18.06.1998**

(51) Int. Cl.$^6$: **B01J 27/198**, C07C 51/215, C07C 57/145

(54) **Verfahren zur Herstellung von Schalenkatalysatoren für die Synthese von Maleinsäureanhydrid durch Gasphasenoxidation**

Preparation process of a coated catalyst for the synthesis of maleic anhydride in the gas phase

Procédé de préparation d'un catalyseur comportant un revêtement pour la synthèse de l'anhydride maléique par oxydation en phase gazeuse

(84) Benannte Vertragsstaaten:
**BE DE ES FR GB IT**

(30) Priorität: **26.06.1997 DE 19727235**

(43) Veröffentlichungstag der Anmeldung:
**26.05.1999 Patentblatt 1999/21**

(73) Patentinhaber:
**Consortium für Elektrochemische Industrie GmbH**
**81379 München (DE)**

(72) Erfinder:
• **Groke, Dirk, Dr.**
**82024 Taufkirchen (DE)**
• **Ruedinger, Christoph, Dr.**
**81479 München (DE)**
• **Eberle, Hans-Juergen, Dr.**
**81477 München (DE)**
• **Bosch, Richard**
**82110 Germering (DE)**
• **Jering, Reinhard**
**80689 München (DE)**

(74) Vertreter:
**Schuderer, Michael, Dr. et al**
**Wacker-Chemie GmbH**
**Zentralabteilung Patente**
**Marken und Lizenzen**
**Hanns-Seidel-Platz 4**
**81737 München (DE)**

(56) Entgegenhaltungen:
WO-A-96/25230          FR-A- 2 245 651
US-A- 4 336 198        US-A- 5 288 880

**Beschreibung**

Die Erfindung betrifft ein Verfahren zur Herstellung von Schalenkatalysatoren für die Gasphasenoxidation von $C_4$-Kohlenwasserstoffen zu Maleinsäureanhydrid, sowie die Verwendung dieser Schalenkatalysatoren in Verfahren zur Gasphasenoxidation von gesättigten oder ungesättigten $C_4$-Kohlenwasserstoffen zu Maleinsäureanhydrid.

Die Herstellung von Maleinsäureanhydrid (= MSA) aus $C_4$-Kohlenwasserstoffen mittels katalytischer Gasphasenoxidation ist seit ca. 20 Jahren bekannt. Dabei werden Katalysatoren auf Basis von Vanadylphosphaten (Vanadylpyrophosphat, Vanadium-Phosphor-Oxide) eingesetzt. Die Herstellung dieser Vanadylphosphate erfolgt über einen Katalysatorvorläufer (Precursor), der auf zwei Reaktionswegen zugänglich ist: 1. Herstellung im wässrigen Medium, 2. Herstellung in organischen Lösungsmittel. Der Precursor wird dann in einem zweiten Schritt vor oder nach der Formgebung entweder im Reaktor (in situ) oder extern in die eigentlich katalytisch aktive Substanz umgewandelt.

Die Umsetzung des $C_4$-Kohlenwasserstoffs am Katalysator erfolgt in verschiedenen Reaktortypen. Zum Einsatz kommen Festbett, Wirbelschicht und auch Riser-Reaktoren, wobei stets die oben aufgeführten Katalysatoren eingesetzt werden. Für den Einsatz in Festbettreaktoren werden Vollkontakte, daS heißt Katalysatoren die ausschließlich aus katalytisch aktiver Komponente bestehen, eingesetzt. Diese Vollkontakte bringen den Nachteil mit sich, daß große Mengen von aktivem Material eingesetzt werden müssen. Aufgrund der meist kompakten Gestalt der Formkörper resultiert ein hoher Druckverlust in den technischen Reaktoren, was zu einem erhöhten Energiebedarf führt. Diese Nachteile können durch den Einsatz von Schalenkatalysatoren überwunden werden. Ferner kommt es durch die geringere Materialmenge bei Schalenkatalysatoren zu einer deutlich reduzierten lokalen Temperaturerhöhung im Katalysator. Dadurch wird die Selektivität des Katalysators positiv beeinflußt. Da bei einem Schalenkatalysator nur die äußere Hülle auf einem inerten Träger aus der aktiven Komponente besteht, führt dies zu einer deutlichen Reduktion des Materialbedarfs. Durch die Auswahl von Katalysatorträgern mit geeigneter Form (z.B. Ringe) läßt sich der Druckaufbau im Reaktor und damit die benötigte Leistung für das Gebläse reduzieren.

Aus der EP-A 72381 ist ein Verfahren zur Herstellung eines Schalenkatalysators für die Gasphasenoxidation von $C_4$-Kohlenwasserstoffen zu MSA beschrieben. Zur Herstellung des Precursors wird eine Lösung von $V_2O_5$, Uranylacetat und $H_3PO_4$ in Isobutanol in Gegenwart von Chlorwasserstoff erhitzt, und der damit erhaltene Precursor durch Abdestillation von Wasser und Isobutanol isoliert. Nach der Trocknung wird der uncalcinierte Precursor auf inerte Träger aufgebracht. Die Aufbringung erfolgt der Gestalt, daß das inerte Trägermaterial in einem ersten Schritt mit Wasser angefeuchtet wird und anschließend der Precursor gegebenenfalls portionsweise, falls notwendig unter weiterem Wasserzusatz, ohne Verwendung eines Binders, aufgebracht wird. Aufgrund der Anfeuchtung des Trägers mit Wasser wird ein poröses Material mit einer gewissen Aufnahmefähigkeit für Wasser eingesetzt. Nach Aussagen dieser Schrift sind für die Herstellung einsatzfähiger Katalysatoren Gehalte an aufgetragenem Material von 50 bis 80 Gew%, bezogen auf das Gewicht des beschichteten Formkörpers, notwendig. Die Aktivierung bzw. Calcinierung des so gewonnenen Materials wird nach der Auftragung des Precursors auf den Trägerkörper an Luft bei einer Temperatur von 400°C für 16 Stunden durchgeführt. Nachteilig ist, daß aufgrund der Auftragung des uncalcinierten Trägers in wässrigem Medium die Katalysatoraktivität unbefriedigend ist.

In der WO-A 96/25230 wird ein Verfahren zur Herstellung von Schalenkatalysatoren für die MSA-Synthese beschrieben, bei dem der Precursor nicht mit Wasser, sondern mit einem organischen Lösungsmittel und optional mit einem Binder versetzt wird und dann auf den Träger aufgetragen wird. Schließlich wird der Trägerkatalysator durch Calcinierung in die katalytisch aktive Form übergeführt. Dabei kann diese Calcinierung sowohl im Reaktor (in situ) als auch extern durchgeführt werden. Der auf diese Weise hergestellte Katalysator enthält 0.02 g/cm$^3$ bis 0.4 g/cm$^3$ (Schüttvolumen) aktive Komponente. Die Schichtdicke der aktiven Komponente beträgt dabei zwischen 0.1 mm und 0.8 mm. Es wurde gefunden, daß bei Auftragung des uncalcinierten Trägers in einem organischen Lösungsmittel aktivere Trägerkatalysatoren erhalten werden, als bei Auftragung in wässriger Suspension. Nachteilig ist allerdings, daß bei Verwendung eines organischen Lösungsmittels dies zu Problemen durch die Explosionsgefahr der sich bei der Verdampfung bildenden Luft-Lösungsmittel-Gemische führt.

Es bestand somit die Aufgabe, ein Verfahren zur Herstellung eines Schalenkatalysatoren für die Synthese von Maleinsäureanhydrid durch Gasphasenoxidation zur Verfügung zu stellen, mit dem sich die Auftragung des Precursors in organischen Lösungsmitteln vermeiden läßt, ohne eine Aktivitätsminderung bei der Auftragung in wässriger Suspension zu erhalten, und falls möglich sogar eine Ausbeutesteigerung erzielen läßt.

Gegenstand der Erfindung ist ein Verfahren zur Herstellung von Schalenkatalysatoren für die Gasphasenoxidation von $C_4$-Kohlenwasserstoffen zu Maleinsäureanhydrid wobei ein Vanadylphosphat-Precursor mit einem V/P-Verhältnis von 1 : 0.5 bis 1 : 2 und einer mittleren Vanadiumoxidationsstufe von 3.9 bis 4.5 in wässrigem oder organischem Medium hergestellt und getrocknet wird und in wässriger Suspension auf Trägerkörper aufgetragen wird, dadurch gekennzeichnet, daß der Vanadylphosphat-Precursor vor der Auftragung auf die Trägerkörper durch mehrstündiges Erhitzen auf eine Temperatur von 200°C bis 500°C calciniert wird.

Die Herstellung des Precursors kann in an sich bekannter Weise erfolgen, beispielsweise nach den in der US-A 4132670, in der EP-A 72381 oder in der DE-A 19645066 beschriebenen Verfahren. Dazu wird eine Vanadium(V)-Verbindung in Gegenwart einer Phosphor(V)-Verbindung mit einem Reduktionsmittel in wässriger oder in organischer Phase reduziert. Geeignete Vanadium(V)-Verbindungen sind beispielsweise Vanadiumpentoxid, Vanadiumoxidhalogenide wie $VOCl_3$, Vanadiumphosphate, Ammoniummetavanadat, Vanadiumpentahalogenide wie $VCl_5$. Vorzugsweise wird Vanadiumpentoxid verwendet. Als Phosphorverbindungen sind Phosphor (V)-Verbindungen wie beispielsweise Phosphorsäure, Phosphorpentoxid, Phosphorpentachlorid oder Phosphorperhalide geeignet. Vorzugsweise wird Phosphorsäure eingesetzt.

Die Vanadium(V)-Verbindung und die Phosphor(V)-Verbindung werden in einem V/P-Atomverhältnis von 1 : 0.5 bis 1 : 2, vorzugsweise 1 : 0.9 bis 1 : 1.3, eingesetzt. Das Molverhältnis von Reduktionsmittel zu Vanadium(V)-Verbindung beträgt 1 : 1 bis 2 : 1, vorzugsweise 1 : 1 bis 1.5 : 1.

Bei der Herstellung in organischer Phase wird vorzugsweise so vorgegangen, daß ein reduzierend wirkendes organisches Lösungsmittel, vorzugsweise Isobutanol eingesetzt wird. Gegebenenfalls werden zusätzliche Reduktionsmittel wie Benzylalkohol, phosphorige Säure oder Chlorwasserstoff zugegeben. Zur Reduktion des Vanadiums wird die Vanadium(V)-Verbindung, vorzugsweise Vanadiumpentoxid, in der organischen Phase, beispielsweise Isobutanol, vorzugsweise im Gemisch mit Reduktionsmittel, beispielsweise Benzylalkohol, suspendiert, und nach Zugabe der Phosphor(V)-Verbindung, beispielsweise ortho-Phosphorsäure, mehrere Stunden, vorzugsweise 2 bis 20 Stunden, unter Rückfluß erhitzt. Das dabei entstehende Reaktionswasser kann als Azeotrop entfernt werden. Nach Abschluß der Reduktion wird die organische Phase, beispielsweise durch Destillation entfernt und der Precursor getrocknet.

Die Herstellung des Precursors in wässriger Phase erfolgt analog. Die Vanadium(V)verbindung, vorzugsweise Vanadiumpentoxid wird zusammen mit der Phosphor(V)verbindung, vorzugsweise ortho-Phosphorsäure, und dem Reduktionsmittel, vorzugsweise Oxalsäure, vorgelegt und mehrere Stunden, vorzugsweise 2 bis 20 Stunden, unter Rückfluß erhitzt. Nach Abschluß der Reduktion wird das Wasser, beispielsweise durch Destillation entfernt, und der Precursor getrocknet.

Nach Abschluß der Reduktion liegt der Vanadylphosphat-Precursor mit einem V/P-Verhältnis von 1 : 0.5 bis 1 : 2 und einer mittleren Vanadiumoxidationsstufe von 3.9 bis 4.5 vor. Der Precursor kann in Form des getrockneten Pulvers oder gegebenenfalls nach Formgebung, beispielsweise Tablettierung oder Granulierung, der Calcinierung Zugeführt werden.

Die Aktivierung des Precursors erfolgt in an sich bekannter Weise durch oxidative Calcinierung, beispielsweise nach den in der WO-A 93/00166 oder in der DE-A 19645066 beschriebenen Verfahrensweisen. Bei der oxidativen Calcinierung wird der Precursor beispielsweise in einem Umluftofen mehrere Stunden, im allgemeinen 2 bis 20 Stunden, bei einer Temperatur von üblicherweise 200°C bis 500°C in Luft erhitzt. Gegebenenfalls kann die Calcinierung stufenweise unter Einhaltung unterschiedlicher Aufheizraten und phasenweise in Gegenwart unterschiedlicher Atmosphäre, beispielsweise Inertgas, vorzugsweise Stickstoff oder Wasserdampf, erfolgen.

Zur Beschichtung der Trägerkörper wird das calcinierte Vanadylphosphat in Wasser suspendiert. Falls notwendig kann ein zusätzlicher mechanischer Zerkleinerungsschritt durchgeführt werden. Der Suspension können dabei, zur Steuerung der Aktivität des fertigen Katalysators, inerte Füllstoffe als Verdünnungsmittel zugesetzt werden. Die inerten Füllstoffe können auch bereits vor der Calcinierung dem getrockneten Precursorpulver zugegeben werden. Als Verdünnungsmittel kommen dabei zum Beispiel $SiO_2$, $TiO_2$, SiC, Graphit in Betracht. Falls inerte Füllstoffe zugesetzt werden, beträgt deren Anteil im allgemeinen 0.5 bis 25 Gew%, bezogen auf den calcinierten Precursor.

Gegebenenfalls können der Suspension auch Promotoren in Form deren wasserlöslicher Verbindungen, z.B. als Chloride, Carbonate, Hydroxide, oder Nitrate, zugefügt werden. Die Promotoren können alternativ dazu auch bereits bei der Präparation des Precursors zugegeben werden, beispielsweise während oder nach der Reduktion. Als Promotor-Elemente können beispielsweise eingesetzt werden: Li, Fe, Mo, Cr, Ce, Zr, Co, Zn, U, Bi. Die Promotoren werden dabei vorzugsweise in solchen Mengen zugegeben, daS die Atomverhältnisse der einzelnen Promotoren zu V jeweils von 0.01 : 1 bis 0.1 : 1 betragen. Es können auch Kombinationen von Promotoren eingesetzt werden, wobei das Atomverhältnis der Summe der eingesetzten Elemente zu Vanadium im Bereich von 0.01 : 1 bis 0.1 : 1 liegen sollte.

Um eine gleichmäßige Verteilung der Inhaltsstoffe der Suspension zu gewährleisten, ist auf eine intensive Durchmischung der Suspension zu achten. Die Viskosität der Suspension muß dabei an die Erfordernisse der Beschichtungsapparatur angepaßt werden. Der so erhaltenen Suspension wird nun ein organischer Binder, vorteilhaft in Form einer wässrigen Dispersion, zugesetzt. Geeignete Bindemittel sind dem Fachmann bekannt. Bevorzugt sind Polyvinylalkohole, Polyvinylacetat oder Copolymere von Vinylacetat mit Vinyllaurat, Ethylen oder Acrylaten, sowie Polyacrylate. Das Bindemittel wird in einer Menge zugesetzt, die für eine ausreichende Haftung der Beschichtung auf dem Träger sorgt. Im allgemeinen sind Mengen von 10 bis 20 Gew% Bindemittel, bezogen auf den Feststoffgehalt der Suspension, ausreichend. Durch die Variation des Gehaltes an organischem Binder kann die Porosität der Beschichtung nach dem Entfernen des Binders im Reaktor beeinflußt werden - mit steigendem Bindemittelgehalt nimmt die Porosität, aufgrund des Ausbrennens des Bindemittels unter Reaktionsbedingungen, zu. Zur Förderung der Porenbildung können gegebenenfalls weitere organische Verbindungen wie Polyethylenglykole oder Stearinsäure zugegeben werden.

Als Trägermaterialien eignen sich beispielsweise Aluminiumoxid, Aluminate, Siliziumcarbid, Siliciumoxid, Silikate, Steatit, Duranit, Porzellan oder Steingut. Im Prinzip können die Träger beliebige Gestalt und Oberflächenstruktur besitzen.

Bevorzugt werden regelmäßig geformte, mechanisch stabile Körper wie Kugeln, Ringe, Sättel, oder wabenförmige oder mit Kanälen versehene Träger, oder sonstige aus der Technik bekannte Formkörper. Um einen geringen Staudruck durch die Katalysatorschüttung im Reaktor zu erhalten, ist es jedoch von Vorteil Strukturen mit einem möglichst großen Anteil an offenem Volumen zu verwenden, beispielsweise Ringe.

Die Abmessungen der Trägerkörper werden vorwiegend von der Dimensionierung des Reaktors bestimmt in welchem die Katalysatoren eingesetzt werden. Bei der MSA-Herstellung kommen im allgemeinen Röhren- oder Rohrbündelreaktoren zur Anwendung. Die Abmessungen der Trägerkörper werden daher durch den inneren Durchmesser der Reaktionsrohre bestimmt und sollten zwischen 1/2 und 1/10 des Innendurchmessers betragen. Vorzugsweise werden Kugeln mit einem Durchmesser von 3 bis 10 mm und Ringe mit einer Höhe von 3 bis 10 mm, einem Außendurchmesser von 4 bis 10 mm und einer Wandstärke von 1 bis 2 mm eingesetzt.

Die Beschichtung der Träger kann in an sich bekannter Weise erfolgen. Nach Vorlegen der Träger in dem Beschichtungsgerät, beispielsweise Sprühcoater oder Dragiertrommel, werden die Trägerkörper auf die zur Beschichtung notwendige Temperatur, im allgemeinen 50°C bis 100°C, gebracht, um die Verdampfung des der Aufschlämmung zugefügten Wasser sicherzustellen. Die Auftragung der aktiven Komponente kann dabei kontinuierlich oder diskontinuierlich erfolgen. Die kontinuierliche Auftragung ist bevorzugt. Aufgrund der Verwendung von Wasser als Suspendiermittel sind keinerlei Maßnahmen für den Explosionsschutz und die Entsorgung von organischen Lösungsmitteln notwendig.

Der calcinierte Precursor wird dabei, gegebenenfalls im Gemisch mit Promotoren und Verdünnungsmittel, in einer solchen Menge auf die Trägerkörper aufgetragen, daß ein Gewichtsanteil der Aktivkomponente von 20 bis 80 Gew%, bezogen auf das Gesamtgewicht des beschichteten Trägers, erreicht wird.

Nach Auftragen der aktiven Komponente können die so erhaltenen Formkörper direkt in einen Reaktor zur Umsetzung von $C_4$-Kohlenwasserstoffen zu MSA eingefüllt werden.

Ein weiterer Gegenstand der Erfindung ist die Verwendung der erfindungsgemäß hergestellten Schalenkatalysatoren in Verfahren zur Gasphasenoxidation von gesättigten oder ungesättigten $C_4$-Kohlenwasserstoffen zu Maleinsäureanhydrid.

Bei der MSA-Herstellung wird der $C_4$-Kohlenwasserstoff zusammen mit einem sauerstoffhaltigen Gas, in Gegenwart des erfindungsgemäß hergestellten Katalysators, vorzugsweise in Festbettreaktoren umgesetzt. Übliche Festbettreaktoren sind beispielsweise Reaktionsrohre, die zu Rohrbündelreaktoren zusammengefaßt und von einem Wärmetauschmedium umgeben sind. Die Reaktionsrohre sind vertikal angeordnet und werden vom Reaktionsgemisch durchströmt. Sie bestehen aus einem gegenüber Wärmetauschmedium, Katalysator, Edukten und Produkten inerten Material, im allgemeinen Stahl, und besitzen im allgemeinen eine Länge von 2000 bis 6000 mm, einen Innendurchmesser von 10 bis 30 mm und eine Wandstärke von 1 bis 4 mm. Als Wärmetauschmedien haben sich in der Praxis eutektische Salzgemische bewährt, beispielsweise eine chloridfreie Schmelze aus Kaliumnitrat und Natriumnitrit. Der Katalysator wird von oben in die Reaktionsrohre eingefüllt und durch in der Nähe der unteren Rohrenden angebrachte Halterungen fixiert.

Geeignete Edukte sind gesättigte oder ungesättigte $C_4$-Kohlenwasserstoffe oder deren Gemische, beispielsweise n-Butan, 1-Buten, 2-Buten (cis und trans), 1,3-Butadien. Vorzugsweise wird n-Butan eingesetzt. Es können aber auch andere Kohlenwasserstoffe mit mindestens vier sich in einer Reihe befindenden C-Atomen eingesetzt werden, beispielsweise Pentan oder (Cyclo)pentene. Das Reaktionsgas besteht üblicherweise aus einem Gemisch aus sauerstoffhaltigem Gas, vorzugsweise Luft, und $C_4$-Kohlenwasserstoff, wobei der Kohlenwasserstoff-Anteil im Gemisch 0.5 bis 10 Vol%, vorzugsweise 1 bis 3 Vol%, betragen kann. Die Reaktionstemperatur beträgt im allgemeinen von 300°C bis 500°C. Die Reaktionsrohre werden vertikal von der Reaktionsmischung durchströmt, wobei die gewünschte Reaktion exotherm abläuft. Die Gasgeschwindigkeit der Reaktionsmischung ist dabei so ausgelegt, daß pro Volumen Katalysator die 500-fache bis 4000-fache Gasmenge pro Stunde durch den Reaktor geleitet wird (GHSV (gas hourly space velocity) = 500 - 4000 h$^{-1}$). Die Reaktionsprodukte verlassen den Reaktor und werden durch nachgeschaltete Rückhaltesysteme (Wäscher, Kondensatoren) aus dem Reaktionsgasstrom entfernt. Als Nebenprodukte fallen neben dem gewünschten Produkt MSA überwiegend CO, $CO_2$ und Spuren von Essig- und Acrylsäure an. Das Rohprodukt wird durch geeignete Methoden (z.B. Destillation) aufgearbeitet.

In einer bevorzugten Ausführungsform wird der mit dem erfindungsgemäß hergestellten Katalysator befüllte Reaktor bei einer Temperatur von 250°C bis 400°C mit sauerstoffhaltigen Gasgemischen angefahren, vorzugsweise bei einer Temperatur von 300°C bis 350°C. Als sauerstoffhaltiges Gasgemisch kann beispielsweise Luft oder ein Butan/Luft-Gemisch mit einer Butan-Konzentration in einem Bereich zwischen 0.25 Vol% bis 2.0 Vol%, vorzugsweise zwischen 0.5 Vol% und 1.5 Vol%, eingesetzt werden. Die Geschwindigkeit der Gasmischung soll dabei von 500 bis 4000 h$^{-1}$ (d.h. 1 Gasgemisch/h / 1 Katalysatorfüllung) liegen. Diese Anfahrphase nimmt im allgemeinen einen Zeitraum von wenigen Stunden, vorzugsweise 0.5 bis 2 Stunden, in Anspruch. Im Anschluß kann die Reaktion unter Einhaltung der obengenannten, bei der MSA-Herstellung üblichen, Reaktionsbedingungen fortgeführt werden.

Bei der erfindungsgemäßen Vorgehensweise zur Herstellung des Schalenkatalysators sind bei der Verwendung von Wasser keine negativen Einflüsse auf die katalytische Aktivität des Katalysators festzustellen, wie sie bei Einsatz des uncalcinierten Precursors gemäß WO-A 96/25230 beobachtet werden. Gleichzeitig bleiben die Vorteile erhalten, die aus der Auftragung der Aktivkomponenten in wässrigem Medium auf den Trägerkörper resultieren: Durch die Verwendung von Wasser als Suspendiermittel wird die Handhabung während des Auftragvorganges deutlich vereinfacht. Die Explosionsgefahr, wie sie bei der Verwendung von organischen Lösungsmittel besteht, kann ausgeschlossen werden. Gleichzeitig führt die Verwendung von Wasser als Suspensionsmittel zu einer Reduktion der Kosten und der Belastung der Umwelt. Die Entsorgung bzw. Verbrennung der bei den aus dem Stand Technik bekannten Verfahren anfallenden Lösungsmitteldämpfe entfällt.

Ferner zeigt es sich bei Anwendung der aus dem Stand der Technik bekannten Verfahren, daß bei der Beschichtung der Träger mit uncalciniertem Material die Haftfähigkeit der aktiven Komponente auf dem Träger bei der notwendigen Calcinierung stark in Mitleidenschaft gezogen wird. Dies führt zu einer geringeren mechanischen Stabilität der Beschichtung nach der Calcinierung, was das Füllen der Reaktionsrohre mit dem binderfreien Katalysator wegen des starken Abriebs erschwert und zu Problemen im Reaktorbetrieb durch einen erhöhten Druckaufbau führen kann. Durch Anwendung des erfindungsgemäßen Verfahrens lassen sich diese Nachteile umgehen, da die Calcinierung vor dem Aufbringen der aktiven Komponente durchgeführt wird, und damit der Binder beim Einfüllen in den Reaktor noch vorhanden ist.

Besonders überraschend wurde ferner gefunden, daß sich die mechanische Stabilität der Beschichtung durch die erfindungsgemäße Vorgehensweise auch nach der Entfernung des Binders während der MSA-Synthese deutlich erhöht ist. Damit ist über die gesamte Betriebsdauer eine gute Haftfähigkeit der aktiven Masse auf dem inerten Trägermaterial gewährleistet. Zusätzlich wird auch das Entleeren der Reaktoren erleichtert. Bei den nach dem Stand der Technik hergestellten Katalysatoren tritt im Unterschied zu dem hier beschriebenen Katalysator eine große Staubbelastung durch den Abrieb der Beschichtung auf.

Die nachfolgenden Beispiele dienen zur weiteren Erläuterung der Erfindung:

Test zur Bestimmung der mechanische Festigkeit der Beschichtung von Schalenkatalysatoren:

Die Stabilität der Beschichtung wurde für die beschichteten Formkörper mit und ohne Binder durch folgenden Versuchsablauf bestimmt:

20 g der beschichteten Formkörper wurden in ein vertikal eingespanntes Glasrohr mit 22 mm Innendurchmesser und einer Länge von 90 cm geleert. Das untere Ende des Glasrohrs war mit einem Becherglas bündig verschlossen. Durch den Aufprall platzte je nach Haftung ein Teil der Beschichtung von den Formkörpern ab. Durch vorsichtiges Abtrennen des abgeplatzten Material wurde der Gewichtsverlust der Formkörper bestimmt. Diese Größe wurde als Maß für die Stabilität bzw. Haftfähigkeit der Beschichtung (Gew% Abrieb) verwendet. Als Maßzahl wurde der Anteil an aktiver Komponente angegeben, der sich bezogen auf die ursprüngliche Menge bei der Durchführung des Tests vom Träger gelöst hat.

Bestimmung der katalytischen Eigenschaften von Schalenkatalysatoren:

Die katalytische Testung der Katalysatoren wurde in folgender Weise vorgenommen:

In einem elektrisch beheizten Röhrenofen wurde in einem Quarzrohr(Innendurchmesser:19 mm) eine 11 cm lange Schüttung aus Katalysatorformkörpern hergestellt (Schüttungsvolumen: 31.2 ml). Die Einwaage an Katalysator wurde registriert. Zusätzlich wurde ein Thermoelement (Ni-CrNi) in der Schüttung plaziert, um eine Messung der Reaktionstemperatur zu ermöglichen. Zur Dosierung der Gase Luft und Butan wurden ein Rotameter bzw. ein Massenfluß-Regler (Fa. Brooks, Typ: 5850E) benutzt. Für die Versuche wurde eine Luftmenge von 600 $ml_n$/min und 5 $ml_n$/min n-Butan entsprechend einer Butan-Konzentration von 0.83 Vol% und einer Gasgeschwindigkeit von 1165 $h^{-1}$ verwendet ($ml_n$ = Gasvolumen im Normzustand bei 0°C, 1 bar in ml). Das gebildete Maleinsäureanhydrid wurde in einer mit Wasser gefüllten Waschflasche über einen bekannten Zeitraum aufgefangen und durch Titration gegen 0.1 n NaOH mit Phenolphthalein als Indikator tritrimetrisch bestimmt. Durch eine entsprechende Ventilschaltung konnte sowohl das Eingangsgasgemisch als auch das Abgas, nach Entfernung der kondensierbaren Anteile, mit Hilfe eines Flammenionisationsdetektors (= FID, umgebauter Gaschromatograph Hewlett-Packard HP 5890 II) analysiert und damit der Umsatz bestimmt werden. Aus der gebildeten MSA-Menge pro Zeit und dem Butanmenge wurde dann die Ausbeute berechnet.

Umsatz, Ausbeute und Selektivität ergeben sich nach folgenden Beziehungen:

$$\text{Umsatz U [\%]} = \text{Int(ein,C}_4) - \text{Int(aus,C}_4) / \text{Int(ein,C}_4)$$
$$\text{Ausbeute A [mol\%]} = n(\text{aus,MSA}) / n(\text{ein,C}_4)$$
$$= 0.5 \times \text{Verbrauch(0.1n NaOH/ml)} / v(\text{ein,C}_4) \times t$$
$$\text{Selektivität S[\%]} = A / U$$

Erläuterung der verwendeten Symbole:

Int(ein/aus, X): Intensität FID-Signal Ein- bzw. Ausgang
n(ein/aus, X): Molzahl der Komponente X am Ein- bzw. Ausgang
$v(\text{ein,C}_4)$: Gasfluß n-Butan in mol/h = $c(\text{ein,C}_4) \times v(\text{ein,gesamt})$ $c(\text{ein,C}_4) = 0.83$ Vol%

$$v(\text{ein,gesamt}) = 27 \text{ mmol/h} = 605 \text{ ml}_n/\text{h}$$

t: Reaktionszeit in h

Da die Volumenänderung bei der Reaktion sich im Bereich von wenigen Prozent bewegt, kann die Ausbeuteberechnung gemäß obiger Gleichung durchgeführt wurden.
Eventuell gebildete Nebenprodukte und deren Konzentration lassen sich durch Analyse des Waschwasser mit Hilfe der Ionenchromatographie bestimmen. Während der Testläufe wurde darauf geachtet, daß die Schüttungstemperatur 480°C nicht überschreitet, da ansonsten eine Schädigung des Katalysators zu befürchten ist. Zur Beurteilung der Katalysatoren wurde die Temperatur des Ofens solange variiert, bis die maximale Ausbeute erreicht wurde. Die Temperatur und der Umsatz bei maximaler Ausbeute wurden notiert.

Herstellung der Schalenkatalysatoren:

Beispiel 1:

Erfindungsgemäße Herstellung eines MSA-Schalenkatalysators

Eine Mischung aus 967 ml (770 g) iso-Butanol und 107 ml (112 g) Benzylalkohol wurde unter Rühren mit 100 g Vanadiumpentoxid in der Kälte versetzt. Die Mischung wurde auf Rückflußtemperatur erhitzt (107°C) und für 3 h unter diesen Bedingungen gehalten. Nach Abkühlen der Mischung um 20°C wurden 121.8 g 106 %ige Phosphorsäure hinzugegeben und anschließend erneut bis zum Rückfluß erhitzt. Nach Ablauf einer weiteren Rückflußzeit von 16 h wurde das Gemisch auf ca. 50°C abgekühlt und filtriert. Der so erhaltene blaue Filterkuchen wurde bei 150°C für 10 h getrocknet und zu Tabletten verpreßt.
Zur Calcinierung wurden die Tabletten in einem Röhrenofen vorgelegt. Vor Beginn des Aufheizvorgangs, wurde der Ofen mit einer Gasmischung von 50 % Stickstoff / 50 % Luft beschickt. Die Ofentemperatur wurde nun bei konstantem Gasfluß erhöht, sodaß nach einer Zeit von 4 h eine Temperatur von 150°C erreicht wurde. Nun wurde die Gaszusammensetzung so geändert, daß ein Gasgemisch aus 50 % Luft und 50 % Dampf erhalten wurde. Die Temperatur wurde nun im Verlauf von 10 h auf 420°C erhöht und für 4 h dort gehalten. Nach Ablauf der Haltezeit wurde der calcinierte Precursor unter Stickstoff auf Raumtemperatur abgekühlt.
800 g des calcinierten Precursors wurden mit 2 l entionisiertem Wasser versetzt und über Nacht gerührt. Die so erhaltene Suspension wurde in ein Glasgefäß überführt, mit 269 g einer Polyvinylacetat-Dispersion (Feststoffgehalt: 50 %) versetzt, auf 3.5 l aufgefüllt und über eine Zeit von 1 h intensiv durch Rühren durchmischt und durch ein 0,5 mm Sieb gegeben.
Nach Einfüllen von 800 g Träger (4 mm Steatit-Kugeln) und Aufheizen der Beschichtungsmaschine (Fa. Glatt, Typ Uniglatt) wurde die Suspension über einen Zeitraum von 4 h aufgesprüht. Nach Abkühlen der Träger auf Raumtemperatur wurden diese entnommen.
Es wurden 1762 g Schalenkatalysator erhalten (Katalysator I). Die aufgebrachte Menge an aktiver Komponente wurde nach oxidativer Entfernung des Binders durch Rückwaage bestimmt. In dem obigen Versuch wurde ein Gehalt an aktiver Komponente (= aktive Masse) von 50 Gew% gefunden. Die Abriebfestigkeit und die katalytischen Daten wurde gemäß den obigen Methode bestimmt und sind in Tabelle 1 zusammengefasst.

Beispiel 2:

Erfindungsgemäße Herstellung eines MSA-Schalenkatalysators (modifizierter Anteil an Aktiv-Komponente)

375 g eines in Tablettenform calcinierten Precursors gemäß Beispiel 1 wurden mit 1 l entionisiertem Wasser versetzt und über Nacht gerührt. Die so erhaltene Suspension wurde in ein Glasgefäß überführt, mit 126 g einer Polyvinylacetat-Dispersion (Feststoffgehalt: 50 %) versetzt, auf 2 l aufgefüllt und über eine Zeit von 1 h intensiv durch Rühren durchmischt und durch ein 0,5 mm Sieb gegeben.

Nach Einfüllen von 1 kg Träger (4 mm Steatit-Kugeln) und Aufheizen der Beschichtungsmaschine (Fa. Glatt, Typ Uniglatt) wurde die Suspension über einen Zeitraum von 2 h aufgesprüht. Nach Aufbringen der gesamten Menge an Suspension wurde die Apparatur mit Wasser nachgespült, um eventuelle in Zuleitungen verbliebene Reste der Suspension zu entfernen. Nach Abkühlen der Träger auf Raumtemperatur wurden diese entnommen.

Es wurden 1454 g beschichtete Träger erhalten (Katalysator II). Die aufgebrachte Menge an aktiver Komponente wurde analog Beispiel 1 bestimmt. Es wurde ein Gehalt an aktiver Komponente (= aktive Masse) von 27.0 Gew% gefunden. Die Testung des Katalysators und die Bestimmung der mechanischen Eigenschaften erfolgt wie oben beschrieben. Die Ergebnisse finden sich in Tabelle 1.

Beispiel 3 (Vergleichsbeispiel) :

Herstellung von MSA-Schalenkatalysatoren gemäß dem Verfahren der WO-A 96/25230

Die Herstellung des Vanadylphosphat-Precursors erfolgte bezüglich des Reduktionsschrittes und der Trocknung analog Bsp. 1. Zur Herstellung der schalenkontakte wurden 200 g des Trägers (4 mm Steatit-Kugeln) in einen sich drehenden zylindrischen Behälter gegeben und mit Hilfe eines warmen Luftstrom auf 50°C aufgeheizt. Es wurde eine Suspension von 200 g Katalysator-Precursors in 300 g iso-Butanol hergestellt und mit Hilfe einer Sprühpistole auf die Oberfläche der Träger aufgebracht. Der beschichtete Katalysator wurde nun in einem nächsten Schritt der Calcinierung unterzogen.

Die Calcinierung erfolgte analog der Verfahrenweise aus Beispiel 1, mit dem Unterschied, daß in dem Röhrenofen bereits beschichteter Schalenkatalysator vorgelegt wurde.

Der so erhaltene Katalysator (Katalysator III) wurde unter den oben aufgeführten Bedingungen auf seine katalytische Leistung getestet. Die Testergebnisse sind in Tabelle 1 zusammengefaßt. Die mechanische Stabilität der Beschichtung wurde ebenfalls gemäß der obigen Methode für den uncalcinierten, calcinierten und den gebrauchten Katalysator ermittelt. Die Daten finden sich ebenfalls in Tabelle 1.

Beispiel 4 (Vergleichsbeispiel):

Herstellung eines MSA-Schalenkatalysator mit dem Verfahren gemäß EP-A 72381

Die Herstellung des Vanadylphosphat-Precursors erfolgte bezüglich des Reduktionsschrittes und der Trocknung analog Bsp. 1. Zur Beschichtung der Träger mit einem Anteil an aktiver Komponente von 50 Gew%, bezogen auf das Gewicht der beschichteten Träger, wurden 50 g Steatit-Kugeln (4 mm Durchmesser) in einem zylindrischen Glasgefäß mit 5 g destilliertem Wasser vermischt. Nachdem sich das Wasser auf dem Träger verteilt hat, wurden 50 g des Precursors in 5 Portionen hinzugegeben. Nach der Zugabe von jeweils 10 g wurde die Apparatur für jeweils weitere 10 min in Bewegung gehalten. Nach der Zugabe der zweiten Portion wurden erneut 4 g Wasser, das man erneut für 10 min einwirken ließ, zugegeben. Nun erfolgte die Zugabe der dritten Portion Precursor. Nach weiteren 15 min, die benötigt wurden, damit das Pulver vollständig von den Trägern aufgenommen wurde, wurden die vierte und fünfte Portion Precursor mit zwischenzeitlichem Aufsprühen von Wasser aufgetragen. Für die Aufnahme der vierten Precursor-Charge wurden 30 min, für die Aufnahme der fünften Portion 40 min benötigt. Die gleichmäßig beschichteten Träger wurden bei 110°C für 16 h getrocknet und anschließend analog der in Beispiel 1 beschriebenen Methode calciniert, mit dem Unterschied, daß in dem Röhrenofen bereits beschichteter Schalenkatalysator vorgelegt wurde. Die Messung der katalytischen Leistung und der mechanischen Stabilität des Katalysators (Katalysator IV) wurde gemäß obiger Methoden durchgeführt. Alle Meßdaten sind in der Tabelle 1 zusammengestellt.

Beispiel 5:

Verwendung von Promotoren bei der Herstellung von Schalenkatalysatoren gemäß Beispiel 1 (erfindungsgemäße Herstellung):

a.) 500 g (= 3.25 mol V) calcinierter Precursor, der analog Beispiel 1 hergestellt wurde, wurden in 750 ml Wasser suspendiert. Zu der Mischung wurden 12.74 g (= 0.065 mol Mo) Ammoniummolybdat ($(NH_4)_2MoO_4$) gegeben, nach dessen Auflösung wurde das Gemisch mit 233.1 g Polyvinylacetat-Dispersion (Festgehalt: 50%) versetzt, gleichmäßig durchmischt und auf eine Gesamtvolumen von 3 l aufgefüllt. Diese Suspension wurde auf inerte Träger (4 mm Kugeln aus Steatit) aufgetragen. Der Beschichtungsprozess war analog zum Beispiel 1.

b.) 500 g (= 3.25 mol V) calcinierter Precursor, der analog Beispiel 1 hergestellt wurde, wurden in 750 ml Wasser suspendiert. Zu der Mischung wurden 4.8 g (= 0.065 mol Li) Lithiumcarbonat ($Li_2CO_3$) gegeben und analog a.) mit 233.1 g Polyvinylacetat-Dispersion durchmischt und auf 3 l aufgefüllt. Der Coat-Prozess wurde mit 4 mm Steatit-Kugeln analog zu Beispiel 1 durchgeführt.

c.) 500 g (= 3.25 mol V) calcinierter Precursor, der analog Beispiel 1 hergestellt wurde, wurden in 750 ml Wasser suspendiert. Zu der Mischung wurden 18.9 g (= 0.065 mol Co) Kobaltnitrat ($Co(NO_3)_2 \cdot 6\ H_2O$) gegeben und analog a.) mit 233.1 g Polyvinylacetat-Dispersion durchmischt und auf 3 l aufgefüllt. Der Beschichtungsprozess wurde mit 4 mm Steatit-Kugeln gemäß Beispiel 1 durchgeführt.

d.) 500 g (= 3.25 mol V) calcinierter Precursor, der analog Beispiel 1 hergestellt wurde, wurden in 750 ml Wasser suspendiert. Zu der Mischung wurden 26.0 g (= 0.065 mol Cr) Chrom(III)nitrat ($Cr(NO_3)_3 \cdot 9\ H_2O$) gegeben und analog a.) mit 233.1 g Polyvinylacetat-Dispersion durchmischt und auf 3 l aufgefüllt. Der Beschichtungsprozess wurde mit 4 mm Steatit-Kugeln gemäß Beispiel 1 durchgeführt.

e.) 500 g (= 3.25 mol V) calcinierter Precursor, der analog Beispiel 1 hergestellt wurde, wurden in 750 ml Wasser suspendiert. Zu der Mischung wurden 14.3 g (= 0.065 mol Zn) Zinkacetat ($Zn(CH_3COO)_2 \cdot 2\ H_2O$) gegeben und analog a.) mit 233.1 g Polyvinylacetat-Dispersion durchmischt und auf 3 1 aufgefüllt. Der Beschichtungsprozess wurde mit 4 mm Steatit-Kugeln gemäß Beispiel 1 durchgeführt.

Beispiel 6:

Einsatz von Verdünnungsmitteln bei der Herstellung von MSA-Schalenkatalysatoren gemäß Beispiel 1 (erfindungsgemäß)

Es wurde eine Suspension ausgehend von 500 g calciniertem Precursor, 100 g Titandioxid (BET-Oberfläche: 8 $m^2$/g) und 233.1 g Polyvinylacetat-Dispersion hergestellt. Das Gesamtvolumen der Suspension betrug 3 l. Der Beschichtungsprozess wurde mit 4 mm Steatit-Kugeln durchgeführt und erfolgt wie in Beispiel 1 beschrieben.

Interpretation der Ergebnisse:

Beim Vergleich der mechanischen Stabilität der verschiedenen Katalysatoren zeigt sich deutlich, daß die nach dem erfindungsgemäßen Verfahren hergestellten Katalysatoren sowohl vor als auch nach Gebrauch den nach bekannten Methoden hergestellten Katalysatoren überlegen sind. So gehen bei den herkömmlich hergestellten Katalysatoren vor Gebrauch im calcinierten Zustand zwischen 12.5 Gew% und 20 Gew% der aktiven Komponente verloren, während bei den erfindungsgemäß hergestellten Katalysatoren kein Abrieb festgestellt wurde. Nach der Benutzung im Reaktor wurden für die bisher bekannten Herstellungsverfahren zwischen 12 Gew% und 27 Gew% Abrieb gegenüber 0 bis 3 Gew% bei den erfindungsgemäß hergestellten Proben beobachtet.

Zum Vergleich der katalytischen Leistungsfähigkeit müssen der Katalysator I und IV bzw. II und III miteinander verglichen werden. Die Zuordnung erfolgt in dieser Art und Weise, da die Aktivität der Katalysatoren von der Menge an aktiver Komponente abhängig ist. Daher ist ein sinnvoller Vergleich nur zwischen Kontakten mit ähnlichem Gehalt an aktiver Komponente möglich. Von den Katalysatoren mit 50 Gew% aktiver Masse zeigt das erfindungsgemäß hergestellte System eine deutlich höhere Ausbeute als der nach den wäßrigen Beschichtungsverfahren gemäß EP-A 72381 hergestellte Katalysator IV (I: 53 mol% Ausbeute. IV: 42 mol% Ausbeute). Die schlechte Leistungsfähigkeit des Katalysators IV zeigt sich auch in der hohen Ofentemperatur bei gleichzeitig geringem Umsatz.

Beim Vergleich der Katalysatoren II und III zeigt sich die Überlegenheit des erfindungsgemäßen Herstellungsverfahrens gegenüber dem Verfahren der WO-A 96/25230 (Beschichtung mit org. Lösungsmittel). Auch hier zeigt sich die deutlich erhöhte Aktivität, der höhere Umsatz und der geringere Abrieb des erfindungsgemäßen Systems.

Zusammenfassend zeigen die durchgeführten Versuche, daß das erfindungsgemäße Herstellungsverfahren den bisher bekannten Methoden zur Herstellung von Schalenkatalysatoren zur Oxidation von $C_4$-Kohlenwasserstoffen zu MSA sowohl hinsichtlich der mechanischen Stabilität als auch in der katalytischen Leistungsfähigkeit deutlich überlegen ist.

TABELLE 1:

Leistungsdaten und mechanische Stabilität der MSA-Schalenkatalysatoren

| Katalysator | Aktive Masse | | Leistungsdaten | | | Abrieb (Gew%) | | |
|---|---|---|---|---|---|---|---|---|
| | Gew% | g/cm3 Schüttung | Ofentem- peratur (°C) | Umsatz (%) | Ausbeute (mol%) | frisch | calciniert | gebraucht |
| I (erf.gemäß) | 50 | 0.41 | 350 | 85.7 | 52.9 | entfällt | 0 | 3 |
| II (erf.gemäß) | 27 | 0.26 | 360 | 94.6 | 56.3 | entfällt | 0 | 0 |
| III (Vergleich) | 25 | 0.20 | 370 | 85.6 | 50.9 | 4 | 12.5 | 27 |
| IV (Vergleich) | 50 | 0.50 | 400 | 73.9 | 42.2 | 1 | 20.2 | 11.7 |

EP 0 917 909 B1

## EP 0 917 909 B1

### Patentansprüche

1. Verfahren zur Herstellung von Schalenkatalysatoren für die Gasphasenoxidation von $C_4$-Kohlenwasserstoffen zu Maleinsäureanhydrid wobei ein Vanadylphosphat-Precursor mit einem V/P-Verhältnis von 1 : 0.5 bis 1 : 2 und einer mittleren Vanadiumoxidationsstufe von 3.9 bis 4.5 in wässrigem oder organischem Medium hergestellt und getrocknet wird und in wässriger Suspension auf Trägerkörper aufgetragen wird, dadurch gekennzeichnet, daß der Vanadylphosphat-Precursor vor der Auftragung auf die Trägerkörper durch mehrstündiges Erhitzen auf eine Temperatur von 200°C bis 500°C calciniert wird.

2. Verfahren nach Anspruch 1, dadurch gekennzeichnet, daß der calcinierte Vanadylphosphat-Precursor im Gemisch mit einem oder mehreren Promotoren aus der Gruppe der wasserlöslichen Verbindungen von Li, Fe, Mo, Cr, Ce, Zr, Co, Zn, U, Bi auf die Trägerkörper aufgetragen wird.

3. Verfahren nach Anspruch 1 oder 2, dadurch gekennzeichnet, daß der calcinierte Vanadylphosphat-Precursor im Gemisch mit einem oder mehreren Verdünnungsmitteln aus der Gruppe $SiO_2$, $TiO_2$, SiC, Graphit auf die Trägerkörper aufgetragen wird.

4. Verfahren nach Anspruch 1 bis 3, dadurch gekennzeichnet, daß der calcinierte Vanadylphosphat-Precursor im Gemisch mit einem organischer Binder auf die Trägerkörper aufgetragen wird.

5. Verfahren nach Anspruch 4, dadurch gekennzeichnet, daß der calcinierte Vanadylphosphat-Precursor im Gemisch mit weiteren organischen Verbindungen wie Polyethylenglykole oder Stearinsäure auf die Trägerkörper aufgetragen wird.

6. Verfahren nach Anspruch 1 bis 5, dadurch gekennzeichnet, daß der calcinierte Vanadylphosphat-Precursor in einer solchen Menge auf die Trägerkörper aufgetragen wird, daß ein Gewichtsanteil der Aktivkomponente von 20 bis 80 Gew%, bezogen auf das Gesamtgewicht des beschichteten Trägers, erreicht wird.

7. Schalenkatalysatoren für die Gasphasenoxidation von $C_4$-Kohlenwasserstoffen zu Maleinsäureanhydrid erhältlich mit den Verfahren nach Anspruch 1 bis 6.

8. Verfahren zur Gasphasenoxidation von gesättigten oder ungesättigten $C_4$-Kohlenwasserstoffen oder deren Gemische in einem mit Katalysator befüllten Reaktor, im Gemisch mit einem sauerstoffhaltigen Gas unter Verwendung eines Schalenkatalysators gemäß Anspruch 7, wobei der Kohlenwasserstoff-Anteil 0.5 bis 3.0 Vol% beträgt, die Reaktionstemperatur von 300°C bis 500°C beträgt, und pro Volumen Katalysator die 500-fache bis 4000-fache Gasgemisch pro Stunde durch den Reaktor geleitet wird.

9. Verfahren nach Anspruch 8, dadurch gekennzeichnet, daß der mit Katalysator befüllte Reaktor in einem vorgeschalteten Schritt bei einer Temperatur von 300°C bis 350°C mit sauerstoffhaltigen Gasgemischen angefahren wird.

10. Verfahren nach Anspruch 9, dadurch gekennzeichnet, daß als sauerstoffhaltiges Gasgemisch Luft oder ein Butan/Luft-Gemisch mit einer Butan-Konzentration in einem Bereich zwischen 0.25 Vol% bis 2.0 Vol% eingesetzt wird.

### Claims

1. Process for producing coated catalysts for the gas-phase oxidation of $C_4$-hydrocarbons to maleic anhydride, in which a vanadyl phosphate precursor having a V/P ratio of from 1:0.5 to 1:2 and a mean vanadium oxidation state of from 3.9 to 4.5 is prepared in an aqueous or organic medium and is dried and applied in aqueous suspension to support bodies, characterized in that the vanadyl phosphate precursor is calcined by heating for a plurality of hours at a temperature of from 200°C to 500°C before application to the support bodies.

2. Process according to Claim 1, characterized in that a mixture of the calcined vanadyl phosphate precursor with one or more promoters selected from the group consisting of water-soluble compounds of Li, Fe, Mo, Cr, Ce, Zr, Co, Zn, U, Bi is applied to the support bodies.

3. Process according to Claim 1 or 2, characterized in that a mixture of the calcined vanadyl phosphate precursor with one or more diluents selected from the group consisting of $SiO_2$, $TiO_2$, SiC and graphite is applied to the support bodies.

4. Process according to any of Claims 1 to 3, characterized in that a mixture of the calcined vanadyl phosphate precursor with an organic binder is applied to the support bodies.

5. Process according to Claim 4, characterized in that a mixture of the calcined vanadyl phosphate precursor with further organic compounds such as polyethylene glycols or stearic acid is applied to the support bodies.

6. Process according to any of Claims 1 to 5, characterized in that the calcined vanadyl phosphate precursor is applied to the support bodies in such an amount that the proportion by weight of the active component is from 20 to 80% by weight, based on the total weight of the coated support.

7. Coated catalyst for the gas-phase oxidation of $C_4$-hydrocarbons to maleic anhydride obtainable by a process according to any of Claims 1 to 6.

8. Process for the gas-phase oxidation of saturated or unsaturated $C_4$-hydrocarbons or mixtures thereof, characterized in that a mixture of the $C_4$-hydrocarbons with an oxygen-containing gas is passed through a reactor charged with a coated catalyst according to Claim 7, the proportion of hydrocarbons is from 0.5 to 3.0% by volume, the reaction temperature is from 300°C to 500°C and the volume of gas mixture passed through the reactor per hour is from 500 to 4000 times the volume of catalyst.

9. Process according to Claim 8, characterized in that the reactor charged with the catalyst is started up in a preceding step at a temperature of from 300°C to 350°C using oxygen-containing gas mixtures.

10. Process according to Claim 9, characterized in that the oxygen-containing gas mixture used is air or a butane/air mixture having a butane concentration in a range from 0.25% by volume to 2.0% by volume.

## Revendications

1. Procédé pour la préparation de catalyseurs supportés pour l'oxydation en phase gazeuse d'hydrocarbures en $C_4$ en anhydride d'acide maléique, un précurseur de phosphate de vanadyle étant préparé et séché en milieu aqueux ou organique avec un rapport V/P de 1:0,5 à 1:2 et un état moyen d'oxydation du vanadium de 3,9 à 4,5 et étant appliqué en suspension aqueuse sur des corps de support, caractérisé en ce que le précurseur de phosphate de vanadyle est calciné à une température de 200 à 500°C par chauffage pendant plusieurs heures avant l'application sur les corps de support.

2. Procédé suivant la revendication 1, caractérisé en ce que le précurseur de phosphate de vanadyle calciné est appliqué sur les corps de support en mélange avec un ou plusieurs promoteurs du groupe des composés solubles dans l'eau de Li, Fe, Mo, Cr, Ce, Zr, Co, Zn, U et Bi.

3. Procédé suivant la revendication 1 ou 2, caractérisé en ce que le précurseur de phosphate de vanadyle calciné est appliqué sur les corps de support en mélange avec un ou plusieurs diluants du groupe $SiO_2$, $TiO_2$, SiC, graphite.

4. Procédé suivant les revendications 1 à 3, caractérisé en ce que le précurseur de phosphate de vanadyle calciné est appliqué sur les corps de support en mélange avec un liant organique.

5. Procédé suivant la revendication 4, caractérisé en ce que le précurseur de phosphate de vanadyle calciné est appliqué sur les corps de support en mélange avec d'autres composés organiques comme les polyéthylèneglycols ou l'acide stéarique.

6. Procédé suivant les revendications 1 à 5, caractérisé en ce que le précurseur de phosphate de vanadyle calciné est appliqué sur les corps de support dans une quantité telle qu'on atteint une proportion en poids des composants actifs de 20 à 80% en poids, sur base du poids total du support revêtu.

7. Catalyseurs supportés pour l'oxydation en phase gazeuse d'hydrocarbures en $C_4$ en anhydride d'acide maléique, que l'on peut obtenir par les procédés suivant les revendications 1 à 6.

8. Procédé pour l'oxydation en phase gazeuse d'hydrocarbures en $C_4$ saturés ou insaturés, ou de leurs mélanges dans un réacteur rempli de catalyseur, en mélange avec un gaz oxygéné en utilisant un catalyseur supporté suivant la revendication 7, la proportion d'hydrocarbure s'élevant de 0,5 à 3,0% en volume, la température de réaction s'élevant de 300°C à 500°C, et le mélange gazeux passé par le réacteur par heure étant de 500 à 4000 fois le volume de catalyseur.

9. Procédé suivant la revendication 8, caractérisé en ce que le réacteur rempli de catalyseur est mis en marche lors d'une étape antérieure à une température de 300°C à 350°C avec des mélanges gazeux oxygénés.

10. Procédé suivant la revendication 9, caractérisé en ce qu'on utilise comme mélange gazeux oxygéné de l'air ou un mélange de butane/air avec une concentration en butane dans un intervalle entre 0,25% en volume et 2,0% en volume.